**Europäisches Patentamt**

(19) **European Patent Office**

**Office européen des brevets**

(11) Numéro de publication: **0 152 716**

**B1**

(12) **FASCICULE DE BREVET EUROPÉEN**

(45) Date de publication du fascicule du brevet: **06.04.88**

(51) Int. Cl.⁴: **A 61 G 5/00**, A 61 F 5/01

(21) Numéro de dépôt: **84440070.5**

(22) Date de dépôt: **18.12.84**

(54) **Siège pour handicapés.**

(30) Priorité: **20.12.83 FR 8320736**

(43) Date de publication de la demande:
**28.08.85 Bulletin 85/35**

(45) Mention de la délivrance du brevet:
**06.04.88 Bulletin 88/14**

(84) Etats contractants désignés:
**AT BE CH DE GB IT LI LU NL SE**

(56) Documents cité:
**DE-A-2 020 525**
**DE-A-2 148 248**
**DE-A-2 150 525**
**FR-A-1 472 654**
**FR-A-2 190 013**
**FR-A-2 242 072**
**FR-A-2 332 745**
**NL-A-7 508 804**
**US-A-3 761 126**
**US-A-3 815 586**
**US-A-4 190 287**

(73) Titulaire: **Mathis, Michel, 7, rue Laennec, F-29118 Benodet (FR)**
Titulaire: **Vautier, Ronan, 9, Allée du Sorbier, F-76520 Franqueville St Pierre (FR)**
Titulaire: **Duhoo, Jacques, Appt.55B Résidence "Les Gerbes d'Or", F-62400 Bethune (FR)**

(72) Inventeur: **Mathis, Michel, 7, rue Laennec, F-29118 Benodet (FR)**
Inventeur: **Vautier, Ronan, 9, Allée du Sorbier, F-76520 Franqueville St Pierre (FR)**
Inventeur: **Duhoo, Jacques, Appt.55B Résidence "Les Gerbes d'Or", F-62400 Bethune (FR)**

(74) Mandataire: **Lepage, Jean- Pierre, Cabinet Lemoine & Associés 12, Boulevard de la Liberté, F-59800 Lille (FR)**

## Description

### Domaine technique

La présente invention concerne un siège pour handicapé inapte à la coordination cerveau-neuro-musculaire, ledit siège étant constitué d'au moins une coque enveloppante recouverte d'un garnissage rembourré en plusieurs parties.

### Problème posé

L'invention s'adresse plus particulièrement aux personnes handicapées incapables de conserver activement la position assise et dont les atteintes entrainent un certain nombre de déformation, qui deviendraient irréversibles si elles n'étaient contrlées.

Le siège de l'invention intéresse particulièrement les personnes atteintes d'infirmités motrices cérébrales qui souffrent de l'incapacité de coordonner, par contrôle cérébral, leur action musculaire.

Ces affections entrainent une très importante variété de troubles dont les principaux sont:

- une athétose, sorte de mouvement ample incontrôlé et amplifié par les stimuli-sensoriels et affectifs.

- souvent, une spasticité, hypertonie incontrôlable de certains muscles paralysés, créant des déformations orthopédiques au niveau des différentes parties du corps concernées par le siège de la présente invention et notamment:

a) une attitude hypercyphosante du rachis et parfois même une scoliose,

b) une position de la tête très en arrière et excentrée par rapport au tronc,

c) au niveau des membres inférieurs, une tendance à la luxation spontanée de hanche aux conséquences très sévères.

- parfois une hypotonie flasque de certains muscles, notamment au niveau des muscles érecteurs du rachis.

- souvent une incontinence.

Le siège de l'invention prévoit de s'appliquer plus particulièrement aux enfants handicapés pour lesquels une posture correcte est essentielle pour favoriser une bonne croissance du squelette et un développement psychique le plus normal possible.

### Etat de la technique et inconvénients

De nombreuses recherches ont été faites depuis longtemps dans ce domaine pour améliorer l'état des handicapés. On peut, à ce sujet, citer quatre brevets français qui sont:

- le brevet 2 329 251 du 27 Octobre 1975,
- le brevet 2 365 317 du 22 Septembre 1976,
- le brevet 2 421 597 du 6 Avril 1978,
- et le brevet 2 442 049 du 21 Novembre 1970.

Le brevet 2 329 251 prévoit un appareillage orthopédique pour le maintien d'un malade en position assise et en position verticale avec un corset cage, un siège coque et des dispositifs de maintien des membres inférieurs. Il prévoit aussi une coque rigide sur l'arrière et les côtés du dossier. Il prévoit un corset se laçant sur l'avant, et une coque en deux parties avec un dispositif du maintien des membres inférieurs.

Cet appareillage a cependant les inconvénients de serrer le buste et de gêner la respiration, ce qui n'est pas nécessaire dans la plupart des cas. La doublure de parement qui est décrite est difficile à démonter. De plus, la coque, en divers éléments, est compliquée à assembler et à démonter, et elle est adaptée à des personnes qui ont atteint leur maximum de croissance, car elle ne peut pas se moduler au fur et à mesure de celle-ci.

Cet appareillage ne comporte, par ailleurs, pas de dispositifs d'amortissements lors des mouvements, notamment les mouvements convulsifs. Enfin, il est très onéreux.

Le brevet 2 365 317 décrit un siège baquet avec piètement, le siège présentant une surface d'appui convexe, l'ensemble étant inclinable.

Les inconvénients de ce dispositif sont inhérents, essentiellement, à sa légèreté et il ne maintient pas le buste, ce qui est nécessaire dans beaucoup de cas. Comme le dispositif précédemment décrit, il ne présente pas de réglage de la doublure et du rembourrage, ni de dispositifs d'amortissement lors des convulsions.

Le brevet 2 421 597 concerne un siège orthopédique simple qui présente la plupart des inconvénients signalés ci-dessus.

Le brevet 2 442 049 concerne une chaise avec une pluralité de sections reliées et réglables pour soutenir les fesses, le dos et la tête. Les différentes sections s'articulent entre elles.

De même que ci-dessus, ce dispositif ne maintient pas correctement le buste et il ne comporte pas de capitonnage. L'assemblage de ses différents éléments est relativement compliqué et, comme précédemment, il ne convient pas à des enfants en cours de croissance car il ne peut pas s'adapter à des modifications de taille. Il ne présente pas non plus de dispositifs d'amortissement lors des mouvements convulsifs et il doit être onéreux.

Ces différents dispositifs présentent en outre l'inconvénient de ne pas maintenir les cuisses écartées et ne pas positionner dans les meilleures conditions possibles, la tête fémorale dana le cotyle. La conformation de ces sièges n'assure pas une bonne rotation de la partie fémorale.

La présente invention est destinée à remédier à ces divers inconvénients.

### Exposé de l'invention

L'invention est caractérisée en ce que le dossier de la coque comporte un soutien dorso-lombaire formé par un renflement vera l'avant à ce niveau, et l'assise de la coque présente des formes concaves latérales conformées au bassin et aux cuisses écartées et un bossage convexe central limitant deux échancrures de l'assise correspondant aux passages des jambes, en ce que les parties du garnissage correspondant à l'assise et au dossier sont appliquées de façon amovible contre la coque tandis que les parties latérales de garrnissage correspondant aux cotés des régions fémorales et thoraciques sont reliées à la coque par des liens élastiques compressibles, amovibles, et réglables, la partie assise du garniture comprenant en outre un bossage au niveau du bossage central de la coque favorisant l'abduction des jambes.

Les liens élastiques sont à base de caoutchouc ou de ressorts que l'on peut plus ou moins comprimer à l'aide d'une tige filetée et d'un écrou. A cet effet, la coque présente des concavités accentuées latéralement pour escamoter lesdits liens élastiques. Suivant une forme préférée de réalisation, le garnissage d'assise et de dossier est constitué d'une coque aux formes complémentaires de l'intérieur du dossier et de l'assise de la coque enveloppante et recouverte d'un garnissage et d'une housse amovible. Ce garnissage est avantageusement réalisé en une seule partie.

Les garnissages de côtés sont, de préférence, constitués de deux plaques fémorales et de deux pince-tailles thoraciques. Les plaques fémorales sont rigides et garnies. Les pince-tailles garnis sont rigides sur l'arrière et flexibles vers l'avant.

De préférence, on prévoit des ceintures élastiques au niveau abdominal et au niveau des cuisses.

### Solution au problème, avantages et résultats industriels

On voit que le siège de l'invention est étudié pour permettre, notamment, aux enfants ayant des problèmes énumérés au début du présent mémoire, de retrouver une attitude anatomique se rapprochant le plus possible de l'attitude normale. Le bossage convexe central de l'assise de la coque et du garnissage permet de placer, dans les meilleures conditions possibles, la position de la tête fémorale dans le cotyle, car il assure un bon soutien au niveau fémoral combiné à la profondeur appropriée de l'assise. Les concavités latérales permettent de donner une meilleure rotation à la partie fémorale. En outre, la forme du siège de la coque et de son garnissage améliore le soutien dorso-lombaire et évite la cyphose.

L'avantage essentiel du dispositif de l'invention est de permettre sa modification modulable et réglable par le système des plaques fémorales latérales et de deux pince-taille thoraciques qu'on peut écarter ou rapprocher plus ou moins par un réglage approprié de l'extérieur de la coque à l'aide des boutons moletés avec les écrous. Ceci permet d'avoir une meilleure approche possible de l'anatomie et de la morphologie de la personne concernée. On peut aussi régler l'abduction des hanches en fonction des besoins. Le réglage des appuis thoraciques se fait en fonction des actions à apporter. Un autre avantage notable du siège de l'invention est la présence du pince-taille qui contrôle la position du bassin et du thorax.

On comprend très bien que le siège de l'invention permette de suivre l'évolution morphologique d'un enfant.

De plus, le garnissage amovible permet de résoudre le problème du confort et de l'hygiène grâce à des housses démontables sur les modules.

En outre, le siège de l'invention permet, grâce à la coque, d'adapter facilement des options importantes et multiples telles qu'un appui-tête ou des appuis de jambes.

Il faut signaler que le siège est conçu de manière à être très stable et que la coque, en une seule pièce, est très solide et rigide. Elle peut s'adapter très facilement sur un fauteuil roulant.

L'invention sera mieux comprise à l'aide de la description suivante qui en donne un exemple non limitatif de réalisation pratique et qui est illustré par les dessins joints.

### Brève description des figures

Dans ces dessins,

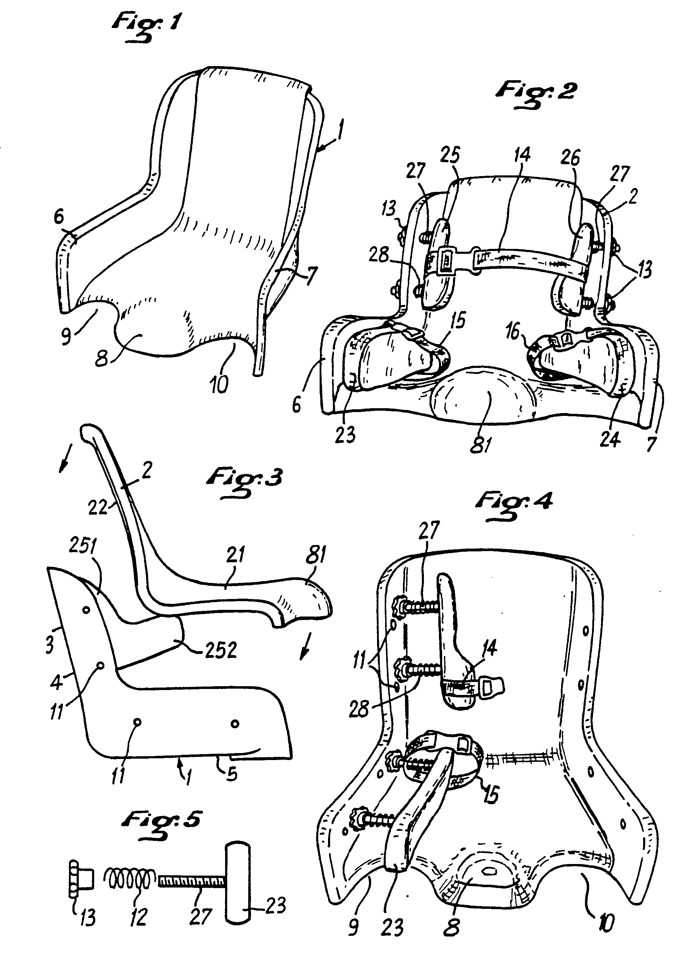

La figure 1 est une vue en perspective de la coque seule.

La figure 2 est une vue en élévation de l'ensemble du siège.

La figure 3 est une vue de profil du siège auquel on adjoint le garnissage en une seule pièce.

La figure 4 est une vue plongeante de l'avant du siège montrant comment on place une plaque fémorale et un pince-taille thoracique.

La figure 5 est une vue de détails montrant un exemple de lien élastique compressible réglable.

### Description d'un mode de réalisation

Le siège de l'invention se compose d'une coque (1) recouverte d'un garnissage rembourré (2).

La coque (1) a un dossier (3) présentant un renflement (4) vers l'avant qui constitue un soutien dorso-lombaire. Elle présente aussi des accoudoirs (6, 7) avec des formes concaves latérales qui correspondent à la forme du bassin et des cuisses écartées. Pour obliger celles-ci à maintenir cette position, on prévoit un bossage convexe central (8) au milieu des deux échancrures (10) qui correspondent au passage des jambes.

Le garnissage (2) comporte, en fait, plusieurs parties. Son assise (21) et son dossier (22) sont appliqués de façon amovible contre la coque (1). L'assise (21) présente, de la même façon, un bossage (81). Les parties latérales du garnissage comportent encore deux plaques fémorales (23, 24) et deux pince-tailles thoraciques (25) et (26). Des plaques fémorales (23, 24) et les pince-tailles (25,26) sont reliées à la coque (1) par des liens élastiques qui sont constitués par des tiges filetées (27, 28) qui traversent la coque (1) par des trous tels que (11) avec interposition de ressorts hélicoïdaux (12) et réglage possible par des boutons moletés (13). Cet ensemble constitue donc une liaison compressible, amovible, et réglable.

Pour disposer les ressorts (12), on accentue les concavités latérales de la coque (11) au voisinage des trous (11).

Le garnissage rembourré (2) est constitué d'une coque rigide aux formes complémentaires de l'intérieur du dossier et de l'assise de la coque (1). Il est recouvert d'une housse amovible avec, éventuellement, l'interposition d'une matière de rembourrage.

Pour des raisons pratiques, on préfère réaliser des plaques fémorales (23, 24) rigides avec un garnissage présentant un léger rembourrage. Pour des raisons similaires, les pince-tailles (25, 26) sont aussi garnies de rembourrage mais sont rigides sur l'arrière en (251) mais flexibles sur l'avant en (252).

Une autre particularité de l'invention réside dans la présence de ceintures élastiques (14) au niveau abdominal et d'autres ceintures (15) et (16) au niveau des cuisses.

**Revendications**

1. Siège pour handicapés inaptes à la coordination cerveau-neuro-musculaire, ledit siège étant constitué d'au moins une coque enveloppante (1) recouverte d'un garnissage rembourré (2) en plusieurs parties, caractérisé en ce que le dossier (3) de la coque comporte un soutien dorso-lombaire formé par un renflement (4) vers l'avant à ce niveau, en ce que l'assise (5) de la coque présente des formes concaves latérales conformées au bassin et aux cuisses écartées et un bossage convexe central (8) limitant deux échancrures de l'assise (9, 10) correspondant aux passages des jambes, en ce que les parties du garnissage (2) correspondant à l'assise (21) et au dossier (22) sont appliquées de façon amovible contre la coque (1) tandis que les parties latérales du garnissage correspondant aux cotés des régions fémorales et thoraciques sont reliées à la coque par des liens élastiques compressibles, amovibles, et réglables, la partie assise du garnissage comprenant en outre un bossage (81) au niveau du bossage central de la coque favorisant l'abduction des jambes.

2. Siège, tel que défini dans la revendication 1, caractérisé par le fait que la coque (1) présente des concavités accentuées latéralement pour escamoter les liens élastiques derrière le garnissage (2).

3. Siège, tel que défini dans l'une ou l'autre des revendications 1 ou 2, caractérisé par le fait que le garnissage (2) d'assise et de dossier est constitué d'une coque aux formes complémentaires de l'intérieur du dossier (3) et de l'assise (5) de la coque enveloppante (1) et recouverte d'un garnissage et d'une housse amovible.

4. Siège, tel que défini dans la revendication 3, caractérisé par le fait que le garnissage (2) d'assise et de dossier est constitué en une partie.

5. Siège, tel que défini dans l'une quelconque des revendications précédentes, caractérisé par le fait que le garnissage de côté est constitué de plaques fémorales (23, 24) et de deux pince-tailles thoraciques (25, 26).

6. Siège, tel que défini dans la revendication 5, caractérisé par le fait que les plaques fémorales (23, 24) sont rigides et garnies.

7. Siège, tel que défini dans la revendication 5, caractérisé par le fait que les pince-tailles (25, 26) garnies sont rigides sur l'arrière et flexibles vers l'avant.

8. Siège, tel que défini dans l'une quelconque des revendications précédentes, caractérisé par le fait que des ceintures (14, 15, 16) élastiques sont prévues au niveau abdominal et au niveau des cuisses.

9. Siège, tel que défini dans l'une quelconque des revendications précédentes, caractérisé par le fait que chaque lien élastique est constitué par un ressort hélicoïdal (12) dans l'axe duquel passe une tige filetée (27) traversant la coque (1) et dont l'enfoncement dans celle-ci est controlé par des boutons moletés (13) réglant la compression des ressorts.

**Patentansprüche**

Stuhl für behindete Personen mit fehler hafter zerebro-neuro-muskulärer Koordination, der aus wenigstens einer den Körper umfassenden und mit einer gepolsterten Bekleidung (2) in mehreren Elementen belegten Schale (1) besteht, dadurch gekennzeichnet, dass die Rückenlehne (3) der Schale eine aus einer nach vorne gerichteten Ausbauchung (4) bestehende Rückenlehne besitz,t dass der Sitz (5) der Schale konkave seitliche Wände aufweist, derer Gestalt mit der des Beckens und der gespreizten Oberschenkel übereinstimmt, sowie eine konvexe Mittelbosse (8), die zwei Ausnehmungen (9, 10) für die Beine begrenzt, dass die mit dem Sitz (21) und der Rückenlehne (22) übereinstimmen den Elemente der Bekleidung (2) wegnehmbar auf der Schale (1) rüsten, während die mit den femoralen und thorakalen Bereichen übereinstimmenden seitlichen Elemente der Bekleidung auf der Schale mittels wegnehmbarer, einstellbarer, elastischer und zusammendrückbarer Verbindungen befestigt sind, wobei der Sitz der Bekleidung eine mit der Mittelbosse der Schale übereinstimmende Bosse (81) aufweist, die die Spreizung der Beine erleichtert.

2. Stuhl nach Anspruch 1, dadurch gekennzeichnet, dass die Schale (1) seitliche konkav ausgebeulte Räume auweist, in denen die elastischen Verbindungen hinter der Bekleidung verborgen werden können.

3. Stuhl nach einem der Ansprüche 1 oder 2, dadurch gekennzeichnet, dass die Sitz- und Rückenbekleidung (2) aus einer Schale besteht, derer Gestalt mit der der Innenseiten der Rückenlehne (3) und des Sitzes (5) der Schale (1) übereinstimmt, und die mit einer Poisterung und einem wegnehmbaren Schonbezug belegt ist.

4. Stuhl nach Anspruch 3, <u>dadurch gekennzeichnet</u>, dass die Sitz- und Rückenbekleidung (2) aus einem Stück gefertigt ist.

5. Stuhl nach irgendeinem der vorhergehenden Ansprüche, <u>dadurch gekannzeichnet</u>, dass die Seitenbekleidung aus femoralen (23, 24) und Taille-Elementen (25, 26) besteht.

6. Stuhl nach Anspruch 5, <u>dadurch gekennzeichnet</u>, dass die femoralen Elemente (23, 24) steft und bekleidet sind.

7. Stuhl nach Anspruch 5, <u>dadurch gekennzeichnet</u>, dass die bekleideten Taille-Elemente (25, 26) am Hinterende stelt und am Vorderende biegsam sind.

8. Stuhl nach irgendeinem der vorhergehenden Ansprüche, <u>dadurch gekennzeichnet</u>, dass elastische Gürtel (14, 15, 16) auf gleicher Höhe des Bauchs und der Oberschenkel vorhanden sind.

9. Stuhl nach irgendeinem der vorhergehenden Ansprüche, <u>dadurch gekennzeichnet</u>, dass jede elastische Befestigungsverbindung aus einer Schraubenfeder (12) besteht, in der eine Gewindespindel (27) montiert ist, die sich durch die Schale (1) soweit erstreckt, wie durch die Einstellung der Federspannung mittels der Rändelknöpfe (13) bestimmt ist.

## Claims

1. Chair for handicapped persons lacking cerebro-neuro-muscular coordination, said chair consisting of at least one body-shaped supporting shell (1) clad with a padded upholstery (2) made of several elements, <u>characterized in that</u> the back (3) of the shell has a dorso-lumbar rest in the form of a front facing bulge (4) at the appropriate height, in that the seat (5) of the shell has concave side walls the shape of which matches that of the pelvis and of the spread thighs, and a convex middle bossage (8) defining in the seat two indentations (9, 10) for the legs, in that the elements of the upholstery (2) corresponding to the seat (21) and the back (22) rest in a detachable manner on the shell (1) while the side elements of the upholstery corresponding to the femoral and thoracic regions are fastened to the shell by means of removable, adjustable, elastic and compressible attachments, the seat part of the upholstery having also a bossage (81) matching the middle bossage of the shell and facilitating the abduction of the legs.

2. Chair according to claim 1, <u>characterized in that</u> the shell (1) has side cavities intended for hiding the elastic attachments behind the upholstery (2).

3. Chair according to any one of claims 1 or 2, characterized in that the seat and back upholstery (2) consists in a shell the shape of which matches that of the internal side of the back (3) and seat (4) of the supporting shell (1) and is wrapped in a loose protection cover.

4. Chair according to claim 3, <u>characterized in that</u> the seat and back upholstery (2) is made in one piece.

6. Chair according to any one of the preceding claims, <u>characterized in that</u> the side lining consists of femoral elements (23, 24) and of two thoracic waist rests (25, 26).

6. Chair according to claim 5, <u>characterized in that</u> the femoral elements (23, 24) are rigid and padded.

7. Chair according to claim 5, <u>characterized in that</u> the padded waist rests (25, 26) are rigid at their back end and flexible at their front end.

8. Chair according to any one of the preceding claims, <u>characterized in that</u> elastic belts (14, 15, 16) are provided at the level of the belly and of the thighs.

9. Chair according to any one of the preceding claims, <u>characterized in that</u> each elastic attachment consists of a coil spring (12) inside which a threaded rod (27) is located, that runs through holes drilled in the shell (1) to such a depth as is determined by means of the knurled knobs (13) intended for setting the tension of the springs.

Fig. 1

Fig. 2

Fig. 3

Fig. 4

Fig. 5